# EUROPEAN PATENT APPLICATION

(11) **EP 4 524 251 A1**
(43) Date of publication of application: **19.03.2025**
(21) Application number: 23803614.9
(22) Date of filing: 11.05.2023
(51) Int. Cl.: C12N 15/864, A61K 35/76, A61K 48/00, A61P 25/00, A61P 25/08, A61P 25/18, A61P 43/00, C12N 7/01

(54) **INHIBITORY NEURON-SPECIFIC PROMOTER**

(30) Priority: 13.05.2022 JP 2022079298
(71) Applicant: National University Corporation Gunma University, Maebashi-shi, Gunma 371-8510 (JP)
(72) Inventor: HIRAI, Hirokazu, Maebashi-shi, Gunma 371-8510 (JP); KONNO, Ayumu, Maebashi-shi, Gunma 371-8510 (JP); MATSUZAKI, Yasunori, Maebashi-shi, Gunma 371-8510 (JP); FUKAI, Yuki, Maebashi-shi, Gunma 371-8510 (JP)
(74) Representative: Gill Jennings & Every LLP
(86) International application number: PCT/JP2023/017785
(87) International publication number: WO 2023/219140

(57) **Abstract**

The present inventors found an inhibitory neuron-specific promoter sequence which, although having a shorter length than the mGAD65 promoter, does not impair any of the promoter activity, specificity for inhibitory neurons, and gene expression efficiency, which is specifically a promoter consisting of DNA having a promoter activity, comprising: a base sequence of a Dlx1 binding site and/or a Dlx2 binding site in a glutamic acid decarboxylase (GAD) promoter; and a base sequence of a region ranging from a 5' end of exon 1 to a transcription start site (TSS) in a glutamic acid decarboxylase (GAD).

## Description

### [Technical Field]

The present invention relates to an inhibitory neuron-specific promoter.

### [Background Art]

Dysfunction of inhibitory neurons, or an abnormal balance of excitation and inhibition in the brain, is deeply involved in the onset of epilepsy, autism, schizophrenia, and the like. Such dysfunction or abnormal balance may be treatable by modulating the function of inhibitory neurons through the transfection with therapeutic genes.

In recent years, adeno-associated virus (AAV) vectors have been widely used in the study of gene expression *in vivo* and are also useful for long-term gene expression in non-dividing cells such as neurons. However, AAV is one of the smallest viruses, and the size of the gene that can be packaged in an AAV vector is as short as about 5.0 kb, including the inverted terminal repeat (ITR) sequences at both ends. Therefore, there is a drawback that there is a limit to the size and number of genes that can be inserted into an AAV vector.

Previously, it has been reported that the distal-less homeobox(Dlx) gene enhancer sequence (which works in combination with a downstream minimal promoter) can be loaded onto an AAV vector to enable the expression of foreign genes specific to inhibitory neurons in the forebrain (Non Patent Literature 1).

The present inventors found that the 2.5 kb upstream of exon 1 of the Gad2 gene, which encodes glutamic acid decarboxylase (GAD) 65 in the mouse genome, acts as an inhibitory neuron-specific promoter (mGAD65 promoter) (Non Patent Literature 2).

The present inventors also found that the mouse-derived Dlx enhancer (mDlx enhancer) has promoter activity specific to inhibitory neurons when combined with a minimal promoter, but it only has promoter activity in the cerebral region (forebrain), whereas the mGAD65 promoter has promoter activity throughout the entire brain (Non Patent Literature 2). Compared to the mDlx enhancer + minimal promoter, the mGAD65 promoter had similar promoter activity in the cerebral region, but was superior in terms of specificity and efficiency for inhibitory neurons.

Meanwhile, the length of the mDlx enhancer + minimal promoter is as short as about 0.7 kb, while the length of the mGAD65 promoter is as long as about 2.5 kb, which occupies about half the packaging limit of the AAV vector, significantly reducing the usefulness of the AAV vector, which is disadvantageous.

### [Citation List]

### [Non Patent Literature]

[Non Patent Literature 1] Nat Neurosci., 2016;19 (12):1743-1749
[Non Patent Literature 2] Mol Brain., 2021;14 (1):33
[Non Patent Literature 3] Nat Biotechnol., 2016;34 (2):204-209
[Non Patent Literature 4] J Comp Neurol., 2018;526 (3):373-396

### [Summary of Invention]

### [Technical Problems]

An objective of the present invention is to obtain an inhibitory neuron-specific promoter sequence which, although having a shorter length than the mGAD65 promoter, does not impair any of the promoter activity, specificity for inhibitory neurons, and gene expression efficiency.

### [Solution to Problem]

The present inventors have conducted diligent investigations in order to solve the problem described above. In the course of the investigation, first, with reference to the mGAD65 promoter described in Non Patent Literature 2, the genomic regions of the mGAD65 promoter and the mGAD67 promoter were cloned. Next, deletion constructs of various regions in each of the mGAD65 and mGAD67 promoters were created, and blood-brain barrier (BBB) permeable AAV (AAV-PHP.eB) vectors expressing green fluorescent protein (GFP) under the control of each promoter sequence were prepared. The obtained AAV vector was administered intravenously to mice, and 3 weeks later, the GFP expression level and expression characteristics for inhibitory neurons were analyzed.

The present inventors focused on the sites in the mGAD65 promoter region where the transcriptional activators Dlx1 and Dlx2 bind. Dlx1 and Dlx2 directly bind to the GAD promoter and activate transcription. The GAD promoter regions to which Dlx1 and Dlx2 have been reported to bind are Region i and Region ii (hereinafter referred to as Ri and Rii, respectively). Thus, these two regions were left and the other regions were deleted from the mGAD65 promoter, or either Ri or Rii was left and the other regions were deleted. The present inventors found that in addition to this deletion treatment, it is particularly important for maintaining high promoter activity to include as a promoter the base sequence of the region from the 5' end of exon 1 to the transcription start site (TSS) (hereinafter also referred to as the E_{TSS} region) in the Gad1 gene encoding GAD67 or the Gad2 gene encoding GAD65, which is not present in the original mGAD65 promoter.

Namely, the present invention is as follows.
[1] A promoter consisting of DNA having a promoter activity, comprising: a base sequence of a Dlx1 binding site and/or a Dlx2 binding site in a glutamic acid decarboxylase (GAD) promoter; and
   a base sequence of a region ranging from a 5' end of exon 1 to a transcription start site (TSS) (E_{TSS} region) in a glutamic acid decarboxylase (GAD).
[2] The promoter according to [1], wherein the glutamic acid decarboxylase (GAD) promoter is a mouse-derived GAD65 promoter (mGAD65 promoter),
   the glutamic acid decarboxylase (GAD) is a mouse-derived glutamic acid decarboxylase 65 (mGAD65),
   the base sequence of the Dlx1 binding site and/or the Dlx2 binding site includes at least one base sequence selected from a base sequence of Region i of the mGAD65 promoter represented by SEQ ID No. 1, a base sequence of Region ii of the mGAD65 promoter represented by SEQ ID No. 2, and a base sequence that is at least 90% identical to any of these base sequences, and
   the base sequence of the region ranging from the 5' end of exon 1 to the transcription start site (TSS) (E_{TSS} region) in mGAD65 is a base sequence represented by SEQ ID No. 3 or a base sequence that is at least 90% identical to the base sequence.
[3] The promoter according to [1] or [2], comprising a base sequence represented by any one of SEQ ID Nos. 4 to 6 or a base sequence that is at least 90% identical to the base sequence represented by any one of SEQ ID Nos. 4 to 6.
[4] The promoter according to [1], wherein the glutamic acid decarboxylase (GAD) promoter is a mouse-derived GAD67 promoter (mGAD67 promoter),
   the glutamic acid decarboxylase (GAD) is a mouse-derived glutamic acid decarboxylase 67 (mGAD67),
   the base sequence of the Dlx1 binding site and/or the Dlx2 binding site includes at least one base sequence selected from a base sequence of Region i of the mGAD67 promoter represented by SEQ ID No. 7, a base sequence of Region ii of the mGAD67 promoter represented by SEQ ID No. 8, and a base sequence that is at least 90% identical to any of these base sequences, and
   the base sequence of the region ranging from the 5' end of exon 1 to the transcription start site (TSS) (E_{TSS} region) in mGAD67 is a base sequence represented by SEQ ID No. 9 or a base sequence that is at least 90% identical to the base sequence.
[5] The promoter according to [1] or [4], comprising a base sequence represented by any one of SEQ ID Nos. 10 to 12 or a base sequence that is at least 90% identical to the base sequence represented by any one of SEQ ID Nos. 10 to 12.
[6] An adeno-associated viral vector or a recombinant virus obtained therefrom for gene expression in an inhibitory neuron, comprising the promoter according to any one of [1] to [5].
[7] The adeno-associated viral vector or the recombinant virus obtained therefrom according to [6], further comprising a target gene arranged under the control of the promoter.
[8] A medicine, comprising the adeno-associated viral vector or the recombinant virus obtained therefrom according to [7].
[9] The medicine according to [8], for treating a central nervous system disease caused by a dysfunction of inhibitory neurons or an abnormal balance of excitation and inhibition.
[10] A method for transfecting a gene into a cell, comprising a step of transfecting a target gene into a target cell *in vitro* by using the adeno-associated viral vector or the recombinant virus obtained therefrom according to [7].
[11] The method according to [10], wherein the target cell is an inhibitory neuron.
[12] A method for treating a central nervous system disease caused by a dysfunction of inhibitory neurons or an abnormal balance of excitation and inhibition, the method comprising administering the adeno-associated viral vector or the recombinant virus obtained therefrom according to [7] into a subject in need thereof.
[13] The adeno-associated viral vector or the recombinant virus obtained therefrom according to [7], which is for use in treating a central nervous system disease caused by a dysfunction of inhibitory neurons or an abnormal balance of excitation and inhibition.
[14] Use of the adeno-associated viral vector or the recombinant virus obtained therefrom according to [7] in producing a medicine for treating a central nervous system disease caused by a dysfunction of inhibitory neurons or an abnormal balance of excitation and inhibition.

### [Advantageous Effects of Invention]

A major problem with the conventional mGAD65 promoter (about 2.5 kb) was that it occupies about half the packaging limit of an AAV vector; however, the present invention makes it possible to provide shortened mGAD65 promoter and mGAD67 promoter. In particular, the compact mGAD65 (cmGAD65, also referred to as mGAD65 (Rii-E_{TSS})) promoter and the compact mGAD67 (cmGAD67, also referred to as mGAD67 (Rii-E_{TSS})) promoter were both successfully shortened significantly to 0.5 kb or less. Furthermore, using an AAV vector carrying the promoter of the present invention has made it possible to express a target gene in inhibitory neurons throughout the central nervous system (CNS) to the same extent as or more potently than the conventional mGAD65 promoter while maintaining specificity for inhibitory neurons and gene expression efficiency.

### [Brief Description of Drawings]

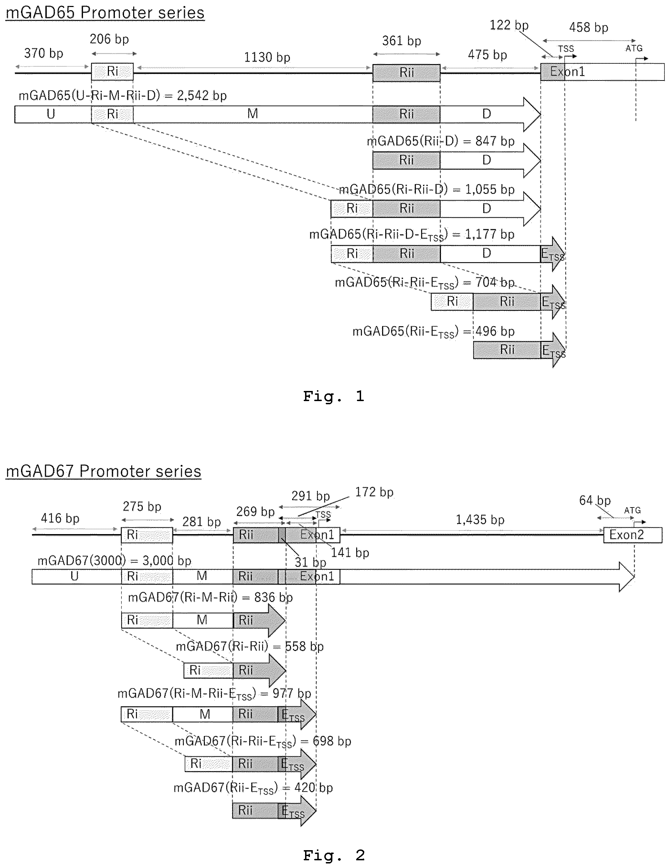

Figure 1 is a schematic diagram of the gene sequences of the shortened mGAD65 promoter series.
Figure 2 is a schematic diagram of the gene sequences of the shortened mGAD67 promoter series.
Figure 3 shows the relative values of GFP fluorescence intensity when gene expression experiments in the cerebral cortex were carried out using various shortened mGAD promoters.
Figure 4 shows the specificity of gene expression when gene expression experiments in the cerebral cortex were carried out using various shortened mGAD promoters.
Figure 5 shows the gene expression efficiency when gene expression experiments were carried out in the cerebral cortex using various shortened mGAD promoters.
Figure 6 shows the mRNA expression level when gene expression experiments in the cerebral cortex were carried out using various shortened mGAD promoters.

### [Description of Embodiments]

### <Promoter>

One embodiment of the present invention is a promoter consisting of DNA having a promoter activity, comprising: a base sequence of a Dlx1 binding site and/or a Dlx2 binding site in a glutamic acid decarboxylase (GAD) promoter; and
a base sequence of a region ranging from a 5' end of exon 1 to a transcription start site (TSS) (E_{TSS} region) in a glutamic acid decarboxylase (GAD).

Glutamic acid decarboxylase (GAD) is an enzyme that synthesizes GABA (gamma-aminobutylic acid) and is known to have two isotypes (GAD65 and GAD67). Their glutamic acid decarboxylase (GAD) promoters are the GAD65 promoter and the GAD67 promoter, respectively.

The animal from which the glutamic acid decarboxylase (GAD) promoter of the present embodiment is derived is not particularly limited as long as it is a mammal having the promoter, and examples thereof include mice, rats, and marmosets. Hereinafter, the mouse-derived GAD65 promoter and GAD67 promoter are also referred to as the mGAD65 promoter and the mGAD67 promoter, respectively.

The respective base sequences contained in the promoter of the present embodiment are preferably contained in the following order: (1) the base sequence of the Dlx1 binding site and/or the Dlx2 binding site, and (2) the base sequence of the E_{TSS} region, but may be in the reverse order as long as they have promoter activity.

The promoter of the present embodiment may be any promoter having the base sequence of the Dlx1 binding site and/or the Dlx2 binding site and the base sequence of the E_{TSS} region. Other regions in the base sequence of glutamic acid decarboxylase (GAD) can also be included as long as they are capable of functioning as a promoter. In addition, any base sequence may be further included for the purpose of enhancing or stabilizing the promoter activity.

The mGAD65 promoter may be, for example, the promoter represented by GenBank no. AB032757, and the mGAD67 promoter may be, for example, the promoter represented by GenBank no. z49978. The sequences of these promoters may include substitutions, deletions, additions, or modifications of about 10% or less of the base sequence (for example, about 1 to 10 bases or about 1 to 100 bases), as long as the promoter of the present embodiment can function as a promoter.

The Dlx1 and/or Dlx2 binding sites are present in the GAD65 and GAD67 promoters, respectively.

Without being particularly limited, for example, when the GAD65 promoter is a promoter represented by GenBank no. AB032757, it may be Region i, which is the region 2294 to 2088 nucleotides upstream on the 5' side from the transcription start site (TSS) of mGAD65, or Region ii, which is the region 958 to 598 nucleotides upstream on the 5' side therefrom.

When the GAD65 promoter is a promoter having a base sequence represented by SEQ ID No. 13, for example, the base sequence of Region i of the mGAD65 promoter may be a base sequence represented by SEQ ID No. 1, and the base sequence of Region ii of the mGAD65 promoter may be a base sequence represented by SEQ ID No. 2. The sequences of these promoters may include substitutions, deletions, additions, or modifications of about 10% or less of the base sequence (for example, about 1 to 10 bases or about 1 to 20 bases), as long as the promoter of the present embodiment can function as a promoter.

In addition, without being particularly limited, for example, when the GAD67 promoter is a promoter represented by GenBank no. z49978, it may be Region i, which is the region 966 to 692 nucleotides upstream on the 5' side from the transcription start site (TSS) of mGAD67, or Region ii, which is the region 410 to 142 nucleotides upstream on the 5' side therefrom.

When the GAD67 promoter is a promoter having a base sequence represented by SEQ ID No. 14, for example, the base sequence of Region i of the mGAD67 promoter may be a base sequence represented by SEQ ID No. 7, and the base sequence of Region ii of the mGAD67 promoter may be a base sequence represented by SEQ ID No. 8. The sequences of these promoters may include substitutions, deletions, additions, or modifications of about 10% or less of the base sequence (for example, about 1 to 10 bases or about 1 to 20 bases), as long as the promoter of the present embodiment can function as a promoter.

When the glutamic acid decarboxylase (GAD) promoter is a mouse-derived GAD65 promoter (mGAD65) in the present embodiment, the base sequence of the Dlx1 binding site and/or the Dlx2 binding site may include at least one base sequence selected from a base sequence of Region i of the mGAD65 promoter represented by SEQ ID No. 1, a base sequence of Region ii of the mGAD65 promoter represented by SEQ ID No. 2, and a base sequence that is at least 90%, preferably at least 95%, and more preferably at least 98% identical to any of these base sequences.

In addition, when the glutamic acid decarboxylase (GAD) is a mouse-derived GAD65 (mGAD65), the promoter may be a promoter in which the base sequence of the region ranging from the 5' end of exon 1 to the transcription start site (TSS) (E_{TSS} region) in mGAD65 is a base sequence represented by SEQ ID No. 3 or a base sequence that is at least 90%, preferably at least 95%, and more preferably at least 98% identical to the base sequence.

In the promoter of the present embodiment, it is preferable that the respective base sequences are contained in the order of (1) the base sequence of Region I and (2) the base sequence of Region ii, but the order may be reversed as long as the promoter of the present embodiment can function as a promoter.

The promoter of the present embodiment may further include other regions in the base sequence of glutamic acid decarboxylase (GAD) before the base sequence of Region i, between the base sequence of Region i and the base sequence of Region ii, or after the base sequence of Region ii, as long as it can function as a promoter. In addition, any base sequence may be further included for the purpose of enhancing or stabilizing the promoter activity.

The promoter of the present embodiment may comprise a base sequence represented by any one of SEQ ID Nos. 4 to 6 or may comprise a base sequence that is at least 90%, preferably at least 95%, and more preferably at least 98% identical to the base sequence represented by any one of SEQ ID Nos. 4 to 6. The promoter of the present embodiment may consist of the base sequence represented by any one of SEQ ID Nos. 4 to 6. The base sequence of SEQ ID No. 4 is a sequence representing Ri-Rii-D-E_{TSS} when the glutamic acid decarboxylase (GAD) promoter is a mouse-derived GAD65 promoter (mGAD65). The base sequence of SEQ ID No. 5 is a sequence representing Ri-Rii-E_{TSS} when the glutamic acid decarboxylase (GAD) promoter is a mouse-derived GAD65 promoter (mGAD65). The base sequence of SEQ ID No. 6 is a sequence representing Rii-E_{TSS} when the glutamic acid decarboxylase (GAD) promoter is a mouse-derived GAD65 promoter (mGAD65). Herein, the region upstream of Ri in mGAD65 may be referred to as U (Upstream), the region between Ri and Rii as M (Middle), and the region downstream of Rii and upstream of the 5' end of exon 1 as D (Downstream).

When the glutamic acid decarboxylase (GAD) promoter is a mouse-derived GAD67 promoter (mGAD67) in the present embodiment, the base sequence of the Dlx1 binding site and/or the Dlx2 binding site may include at least one base sequence selected from a base sequence of Region i of the mGAD67 promoter represented by SEQ ID No. 7, a base sequence of Region ii of the mGAD67 promoter represented by SEQ ID No. 8, and a base sequence that is at least 90%, preferably at least 95%, and more preferably at least 98% identical to any of these base sequences.

In addition, when the glutamic acid decarboxylase (GAD) is a mouse-derived GAD67 (mGAD67), the promoter may be a promoter in which the base sequence of the region ranging from the 5' end of exon 1 to the transcription start site (TSS) (E_{TSS} region) in mGAD67 is a base sequence represented by SEQ ID No. 9 or a base sequence that is at least 90%, preferably at least 95%, and more preferably at least 98% identical to the base sequence.

In the promoter of the present embodiment, it is preferable that the respective base sequences are contained in the order of (1) the base sequence of Region I and (2) the base sequence of Region ii, but the order may be reversed as long as the promoter of the present embodiment can function as a promoter.

The promoter of the present embodiment may further include other regions in the base sequence of glutamic acid decarboxylase (GAD) before the base sequence of Region i, between the base sequence of Region i and the base sequence of Region ii, or after the base sequence of Region ii, as long as it can function as a promoter. In addition, any base sequence may be further included for the purpose of enhancing or stabilizing the promoter activity.

In the promoter of the present embodiment, Region ii and the E_{TSS} region may overlap in part, i.e., the base sequence of Region ii and the base sequence of the E_{TSS} region may partially have a shared portion. When the glutamic acid decarboxylase (GAD) promoter is a mouse-derived GAD67 promoter (mGAD67), it is preferable that Region ii and the E_{TSS} region overlap in part, i.e., it is preferable that the base sequence of Region ii and the base sequence of the E_{TSS} region partially have a shared portion.

The promoter of the present embodiment may comprise a base sequence represented by any one of SEQ ID Nos. 10 to 12 or may comprise a base sequence that is at least 90%, preferably at least 95%, and more preferably at least 98% identical to the base sequence represented by any one of SEQ ID Nos. 10 to 12. The promoter of the present embodiment may consist of the base sequence represented by any one of SEQ ID Nos. 10 to 12. The base sequence of SEQ ID No. 10 is a sequence representing Ri-M-Rii-E_{TSS} when the glutamic acid decarboxylase (GAD) promoter is a mouse-derived GAD67 promoter (mGAD67). The base sequence of SEQ ID No. 11 is a sequence representing Ri-Rii-E_{TSS} when the glutamic acid decarboxylase (GAD) promoter is a mouse-derived GAD67 promoter (mGAD67). The base sequence of SEQ ID No. 12 is a sequence representing Rii-E_{TSS} when the glutamic acid decarboxylase (GAD) promoter is a mouse-derived GAD67 promoter (mGAD67). Herein, the region upstream of Ri in mGAD67 may be referred to as U (Upstream), the region between Ri and Rii as M (Middle), and the region downstream of Rii and upstream of the 5' end of exon 1 as D (Downstream).

The method for producing the promoter of the present embodiment is not particularly limited, and it can be produced by gene recombination techniques known to those skilled in the art.

### <Vector or Recombinant Virus Obtained therefrom>

Another embodiment of the present invention is an adeno-associated viral vector for gene expression in an inhibitory neuron or a recombinant virus obtained therefrom, comprising the above promoter.

It is publicly known to those skilled in the art that there is a plurality of serotypes of adeno-associated viruses (AAV), and the serotype of AAV from which the AAV vector is derived in the present embodiment is not limited as long as it is capable of expressing a target gene in an inhibitory neuron.

The vector of the present embodiment may further comprise a target gene placed under the control of the above promoter, i.e., the target gene linked to the promoter. A type of target gene linked to the promoter is not particularly limited, and fluorescent protein gene such as GFP (comprising enhanced type), a Cre recombinase gene, marker genes such as luciferase, chloramphenicol acetyltransferase, and lacZ, a protein gene that senses production of intracellular second messengers such as Ca²⁺ and cyclic AMP and emits fluorescent light, a light-activated protein gene, a chemical genetics tool such as DREADD, and a toxin gene, can be exemplified. Further, the target gene may be a gene that is used for treating a central nervous system disease caused by a dysfunction of inhibitory neurons or an abnormal balance of excitation and inhibition.

Examples of a central nervous system disease caused by a dysfunction of inhibitory neurons or an abnormal balance of excitation and inhibition can include epilepsy, autism, and schizophrenia. The gene that has a therapeutic effect against these diseases can be selected according to the type of diseases to be treated and the like.

The vector of the present embodiment may be such that these target genes were preliminarily under the control of the above promoter, or a multicloning site for incorporating these target genes as necessary is arranged downstream of the promoter.

The vector of the present embodiment may comprise other functional genes, for example, it may further comprise a woodchuck hepatitis virus post-transcriptional regulatory element (WPRE) and/or a polyadenylation signal (poly A) sequence.

WPRE prevents read-through of the poly A sequence, promotes RNA processing and maturation, and has a function of increasing RNA transportation out of nucleus thereof. WPRE also functions to promote vector packaging by acting on the viral genomic transcript in packaging cells, allowing the viral titer to be increased. Moreover, WPRE promotes the maturation of mRNA produced by the internal promoter of the vector and therefore has a function of enhancing the expression of target genes in transfected target cells.

The vector may contain a selection marker. The "selection marker" is referred to as a genetic element that provides a selectable phenotype for the cell into which the selection marker was transfected and is generally a gene imparting resistance for the chemical reagent in which a gene product inhibits cell growth or kills cells. Specifically, for example, Neo gene, Hyg gene, hisD gene, Gpt gene, and Ble gene are comprised. Drugs useful for selecting the presence of the selection marker comprise, for example, G418 for Neo, hygromycin for Hyg, histidinol for hisD, xanthine for Gpt, and bleomycin for Ble.

The vector of the present embodiment may be composed of inverted terminal repeat (ITR) sequences at both ends of the expression unit containing base sequences of the above primer, target gene, WPRE, poly A, and the like.

The vector of the present embodiment may be a vector consisting of the base sequences represented by SEQ. ID Nos. 15 to 20 alone, or it may be a vector further comprising a base sequence other than the base sequences represented by SEQ. ID Nos. 15 to 20. Further, provided that the promoter and the target gene are functional, the vector may be a vector comprising a base sequence that is at least 90% identical to any of the base sequences represented by SEQ. ID Nos. 15 to 20, it may be preferably a vector comprising a base sequence that is at least 95% identical to any of the base sequences represented by SEQ. ID Nos. 15 to 20, and even more preferably a vector comprising a base sequence that is at least 98% identical to any of the base sequences represented by SEQ. ID Nos. 15 to 20. The base sequences represented by SEQ ID Nos. 15 to 20 are shown in the form of a sequence beginning with the promoter base sequence and going around the plasmid from the 5' end to the 3' end.

The method for incorporating the various genes above into the AAV vector sequence can be carried out by methods publicly known to those skilled in the art. Although not particularly limited, for example, various genes can be incorporated into the vector sequence by a method using restriction enzyme treatment.

As a gene transfection method, a method using publicly known AAV vectors can be employed.

Specifically, a pAAV plasmid, a pRC plasmid, or a helper plasmid is transfected into packaging cells such as HEK293 cells, which are then cultured for a predetermined period of time to form recombinant viruses. Thereafter, the recombinant virus particles can be recovered from the culture supernatant. The culture supernatant containing the recombinant virus particles can be directly used as a recombinant virus. Alternatively, it may be used as a recombinant virus after being subjected to a concentration and/or purification step. The recombinant virus may be used in a state dissolved in a solvent as a recombinant virus solution. The recombinant virus thus obtained can be used to infect target inhibitory neurons.

### <Medicine>

Another embodiment of the present invention is a medicine comprising any of the above adeno-associated virus vectors or a recombinant virus obtained therefrom.

The medicine of the present embodiment can be used for treating a central nervous system disease caused by a dysfunction of inhibitory neurons or an abnormal balance of excitation and inhibition. Examples of a central nervous system disease caused by a dysfunction of inhibitory neurons or an abnormal balance of excitation and inhibition can include epilepsy, autism, and schizophrenia.

The amount of active ingredients in the medicine of the present embodiment is not particularly limited as long as the therapeutic effect can be obtained and can be appropriately determined according to the type of disease, degree of disease, patient age, body weight, expression efficiency, or the like.

The administration and dosage of the medicine of the present embodiment are not particularly limited as long as the therapeutic effect can be obtained and can be appropriately determined according to the type of disease, degree of disease, patient age, body weight, expression efficiency, or the like.

The medicine of the present embodiment may contain other active ingredients, and may be combined for use with, for example, therapeutic agents for treatment of diseases of the central nervous system.

Other embodiments of the present invention include, for example, the following:
a method for treating a central nervous system disease caused by a dysfunction of inhibitory neurons or an abnormal balance of excitation and inhibition, the method comprising administering any of the above adeno-associated viral vectors or a recombinant virus obtained therefrom into a subject in need thereof;
any of the above adeno-associated viral vectors or a recombinant virus obtained therefrom for use in treating a central nervous system disease caused by a dysfunction of inhibitory neurons or an abnormal balance of excitation and inhibition; and
use of any of the above adeno-associated viral vectors or a recombinant virus obtained therefrom in producing a medicine for treating a central nervous system disease caused by a dysfunction of inhibitory neurons or an abnormal balance of excitation and inhibition.

### <Gene Transfection Method>

Another embodiment of the present invention is a method for transfecting a target gene into a target cell *in vitro* by using any of the above adeno-associated viral vectors or a recombinant virus obtained therefrom. However, a method for transfecting any of the above adeno-associated virus vectors or a recombinant virus obtained therefrom into a target cell in non-human animals *in vivo* is also comprised.

The target cell into which the adeno-associated viral vector or the recombinant virus obtained therefrom is transfected is an inhibitory neuron.

The method for transfecting the adeno-associated viral vector or the recombinant virus obtained therefrom into the target cell is not particularly limited, however, is preferably a method based on the infection ability that the adeno-associated viral vector or the recombinant virus obtained therefrom inherently possesses.

### [Examples]

The present invention will be described in more detail by way of the following Examples, however, it goes without saying that the scope of the invention is not limited only to Examples.

### <Method>

### [Promoter]

A plasmid having a template sequence described below was cleaved with a restriction enzyme (the plasmid used in the Examples has an XhoI site upstream of the promoter and an AgeI site downstream of the promoter), and a necessary region of the template plasmid (expected to function as a promoter) was amplified with forward and reverse primers designed to match the cleavage surface, and an InFusion reaction, which is a method known to those skilled in the art, was performed as necessary, thereby preparing the plasmid. The promoter sequence was confirmed by a sequencing reaction using a BigDye (trademark)Terminator v3.1 Cycle Sequencing Kit (Thermo Fisher Scientific; Waltham, MA, USA) or a SupreDye (trademark) Cycle Sequencing Kit (EdgeBio; San Jose, CA, USA) and then using an Applied Biosystems SeqStudio Genetic Analyzer (Thermo Fisher Scientific; Waltham, MA, USA). The thermal cycling program used in the sequencing reaction was carried out with an initial denaturation step at 96°C for 2 min, followed by 25 cycles of 96°C for 10 sec, 50°C for 5 sec, and 60°C for 4 min. The specific production method is as follows.

mGAD65 (Ri-Rii-D-E_{TSS}) represented by SEQ ID No. 4 was prepared by carrying out DNA amplification of Ri-Rii-D from the template sequence represented by SEQ ID No. 21 using forward and reverse primers (SEQ ID Nos. 22 and 23), carrying out DNA amplification of E_{TSS} from the template sequence represented by SEQ ID No. 24 using forward and reverse primers (SEQ ID Nos. 25 and 26), and then fusing these DNAs by an InFusion reaction.

mGAD65 (Ri-Rii-E_{TSS}) represented by SEQ ID No. 5 was prepared by carrying out DNA amplification of Ri-Rii from the template sequence represented by SEQ ID No. 27 using forward and reverse primers (SEQ ID Nos. 28 and 29), carrying out DNA amplification of E_{TSS} from the template sequence represented by SEQ ID No. 24 using forward and reverse primers (SEQ ID Nos. 25 and 26), and then fusing these DNAs by an InFusion reaction.

mGAD65 (Rii-E_{TSS}) represented by SEQ ID No. 6 was prepared by carrying out DNA amplification of Rii-E_{TSS} from the template sequence represented by SEQ ID No. 30 using forward and reverse primers (SEQ ID Nos. 31 and 32).

mGAD67 (Ri-M-Rii-E_{TSS}) represented by SEQ ID No. 10 was prepared by carrying out DNA amplification of Ri-M-Rii-E_{TSS} from the template sequence represented by SEQ ID No. 33 using forward and reverse primers (SEQ ID Nos. 34 and 35).

mGAD67 (Ri-Rii-E_{TSS}) represented by SEQ ID No. 11 was prepared by carrying out DNA amplification of Ri from the template sequence represented by SEQ ID No. 36 using forward and reverse primers (SEQ ID Nos. 37 and 38), carrying out DNA amplification of Rii-E_{TSS} from the template sequence represented by SEQ ID No. 39 using forward and reverse primers (SEQ ID Nos. 40 and 41), and then fusing these DNAs by an InFusion reaction.

mGAD67 (Rii-E_{TSS}) represented by SEQ ID No. 12 was prepared by carrying out DNA amplification of Rii-E_{TSS} from the template sequence represented by SEQ ID No. 42 using forward and reverse primers (SEQ ID Nos. 43 and 44).

### [Viral Vector and Virus]

The viral vector used was the BBB-penetrating AAV-PHP.eB (Non Patent Literature 3). The expression plasmid pAAV, which contains a polyadenylation signal (poly A) sequence and a woodchuck hepatitis virus post-transcriptional regulatory element (WPRE), was designed to express GFP under the control of the mouse genomic region upstream of the mouse Gad2 gene, which encodes glutamic acid decarboxylase (GAD) 65. The shortened mGAD65 promoter was inserted upstream of the GFP gene at the XhoI and AgeI restriction enzyme sites in the pAAV plasmid. pAAV-PHP.eB was constructed based on the pAAV2/9 plasmid provided by Dr. James M. Wilson of the University of Pennsylvania (Non Patent Literature 3).

The recombinant single-stranded AAV-PHP.eB vector was produced by co-transfecting HEK293T cells (HCL4517; Thermo Fisher Scientific; Waltham, MA, USA) with three plasmids (i.e., the above expression plasmid, pHelper (Stratagene, La Jolla, CA, USA), and a packaging plasmid (pAAV-PHP. eB)) by a method known to those skilled in the art.

Briefly, the procedures are explained. First, HEK293T cells cultured in Dulbecco's modified Eagle's medium (D-MEM; D5796-500Ml, Sigma-Aldrich, St. Louis, MO, USA) supplemented with 8% fetal bovine serum (Sigma-Aldrich) were transfected with three plasmids (expression plasmid [pAAV/shortened mGAD65 promoter-EGFP-WPRE-SV40], pHelper (Stratagene, La Jolla, CA, USA), and a packaging plasmid (pAAV-PHP.eB)) using polyethyleneimine. Six days after transfection, viral particles were harvested from the culture medium and concentrated by precipitation with 8% polyethylene glycol 8000 (Sigma-Aldrich) and 500 mM sodium chloride. Precipitated AAV-PHP.eB particles were resuspended in D-PBS and purified by continuous gradient centrifugation of iodixanol (OptiPrep; Axis-Shield Diagnostics, Dundee, Scotland). The resulting virus solution was further concentrated in D-PBS using Vivaspin 20 (100,000 MWCO PES, Sartorius, Gottingen, Germany). The genomic titer of the viral vector was determined by real-time quantitative PCR with Power SYBR Green qPCR Mix (TOYOBO Co., Ltd., Osaka, Japan) by using the primers 5'-CTG TTG GGC ACT GAC AAT TC-3' (SEQ ID No. 45) and 5'-GAA GGG ACG TAG CAG AAG GA-3' (SEQ ID No. 46) targeting the WPRE sequence. An expression plasmid was used as a standard.

### [Animals]

Wild-type mice and VGAT-tdTomato mice on a C57BL/6 background were used (Non Patent Literature 4). Animal care and treatment were carried out in accordance with the Act on Welfare and Management of Animals and the Guidelines for Proper Conduct of Animal Experiments issued by the Science Council of Japan. In addition, when conducting animal experiments, efforts were made to minimize animal suffering and reduce the number of animals used.

### [Intravenous Injection]

7- to 14-week-old mice were used. After deep anesthesia by intraperitoneal injection of ketamine (100 mg/kg BW) and xylazine (10 mg/kg BW), 100 µL of AAV-PHP.eB (5.0 × 10¹² vg/mL) was injected into the orbital venous plexus for 30 to 40 sec using a 0.5-mL syringe with a 30-gauge needle (08277; NIPRO CORPORATION, Osaka, Japan). Three weeks after AAV-PHP.eB vector injection, mice were deeply anesthetized and perfused transcardially with 4% paraformaldehyde in phosphate buffer (pH 7.4). The brains were removed and immersed with 4% paraformaldehyde in 0.1 M phosphate buffer for 6 hours at 4°C.

### [Statistical analysis]

Statistical analysis was performed using GraphPad PRISM version 7 (GraphPad Software, San Diego, CA, USA). Data are expressed as mean ± SEM.

### <Example 1: Relative GFP Fluorescence Intensity in Cerebral Cortex>

Bright-field images and native GFP fluorescence images of the whole brain were obtained using a fluorescent stereomicroscope (VB-700; KEYENCE CORPORATION, Osaka, Japan). To measure GFP fluorescence intensity in the cerebral cortex, the edges of the corresponding portions of the brain, excluding the olfactory bulb, superior and inferior tubercles, and brainstem, were traced on bright-field images. GFP fluorescence intensity within the boxed areas was measured using ImageJ software (Fiji). Autofluorescence was measured in the brains of eight VGAT-tdTomato mice that had not been injected with an AAV vector, and the average value thereof was subtracted from the GFP fluorescence value of all samples. Additionally, data were normalized to the average value from the cerebral cortex of VGAT-tdTomato mice transduced by administered AAV-PHP.eB, which expresses GFP under the control of a shortened mGAD65 promoter. VGAT-tdTomato mice express tdTomato specifically in inhibitory neurons throughout the central nervous system (CNS), which makes it possible to determine whether the transfected (GFP-expressing) cells are inhibitory GABAergic neurons without performing immunohistochemistry.

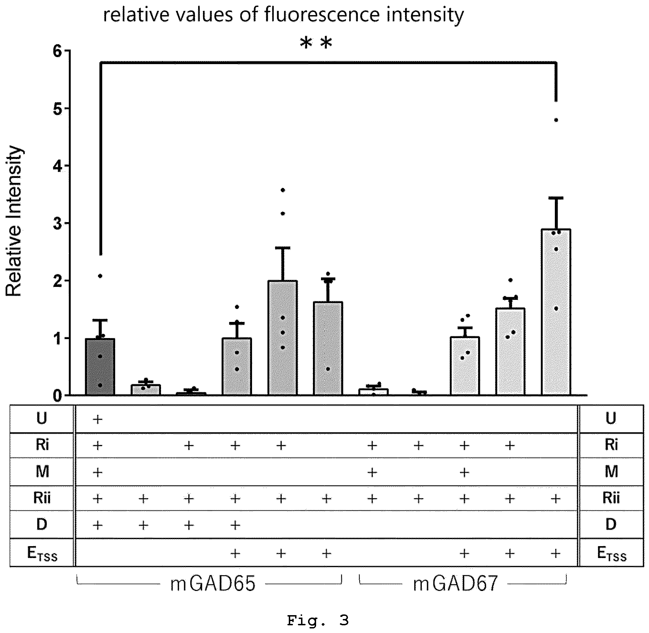

The results are shown in Figure 3 (Mean ± SEM (standard error) (n = 3 to 5), **: P < 0.01, 1-way ANOVA, Dunnett's test). The GFP fluorescence intensity in the case of using shortened mGAD65 promoters or shortened mGAD67 promoters was measured. Some of the shortened mGAD65 promoters showed similar or stronger GFP expression than the original mGAD65 promoter, but there was no significant difference in any case (shown as mGAD65(Ri-Rii-D-E_{TSS}), mGAD65(Ri-Rii-E_{TSS}), and mGAD65(Rii-E_{TSS}) in Figure 3). Meanwhile, some of the promoters showed similar or stronger GFP expression than the original mGAD65 promoter (indicated as mGAD67 (Ri-M-Rii-E_{TSS}), mGAD67 (Ri-Rii-E_{TSS}), and mGAD67 (Rii-E_{TSS}) in Figure 3). In particular, a significant increase in fluorescence intensity was observed in cmGAD67 (mGAD67 (Rii-E_{TSS})), the shortest mGAD67 promoter designed.

### <Example 2: Specificity and Efficiency of Gene Expression in Cerebral Cortex>

To examine the specificity and efficiency of gene expression in the case of using shortened mGAD65 promoters, fluorescent images of GFP and tdTomato were obtained from sagittal brain sections (thickness: 50 µm) using a fluorescence microscope (BZ-X800; KEYENCE CORPORATION) with a 20× objective lens. The numbers of GFP (+) and/or tdTomato (+) cells were counted manually.

The specificity and efficiency of gene expression in inhibitory neurons were examined when various shortened mGAD65 promoters were used. Confocal microscopy image analysis confirmed that GFP and tdTomato fluorescence overlapped in some neurons in VGAT-tdTomato mice treated with AAV-PHP.eB, which expresses GFP under the control of various shortened mGAD65 promoters.

First, a quantitative analysis of gene expression specificity was performed using the following formula: Specificity (%)=(Number of GFP- and tdTomato-co-labeled cells/Total number of GFP-positive cells) × 100. This formula represents the ratio of VGAT-positive cells and cells with consistent GFP expression per AAV vector-derived GFP-expressing cell (cells expressing GFP only are excluded), i.e., the ratio at which the AAV vector specifically expresses GFP in inhibitory neurons.

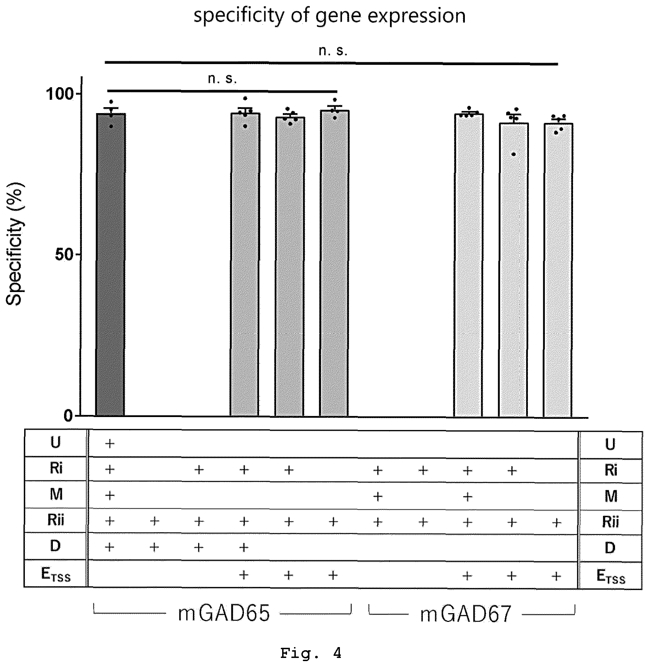

The results of quantitative analysis of gene expression specificity are shown in Figure 4 (Mean ± SEM (standard error) (n = 4 to 5), n.s.: Not Significant, 1-way ANOVA, Dunnett's test). It was shown that not less than 95% of cells expressing GFP under the control of the original mGAD65 promoter were co-labeled with tdTomato (95.4% ± 1.00%, n = 4 to 5). In addition, it was shown that the specificity of gene expression was 95.3% ± 1.02% when the shortest cmGAD65 among the shortened mGAD65 promoters (namely, mGAD65 (Rii-E_{TSS})) was used, and the specificity of gene expression was 91.6% ± 0.97% when the shortest cmGAD67 among the shortened mGAD67 promoters (namely, mGAD67 (Rii-E_{TSS})) was used.

Next, a quantitative analysis of gene expression efficiency was performed using the following formula: Expression efficiency (%) = (Number of GFP- and tdTomato-co-labeled cells/total number of tdTomato-positive cells) × 100

This formula represents the ratio of VGAT-positive cells and cells with consistent GFP expression per VGAT-positive cell (cells that are only VGAT-positive are excluded), i.e., the ratio indicating the efficiency with which the AAV vector expresses GFP in inhibitory neurons.

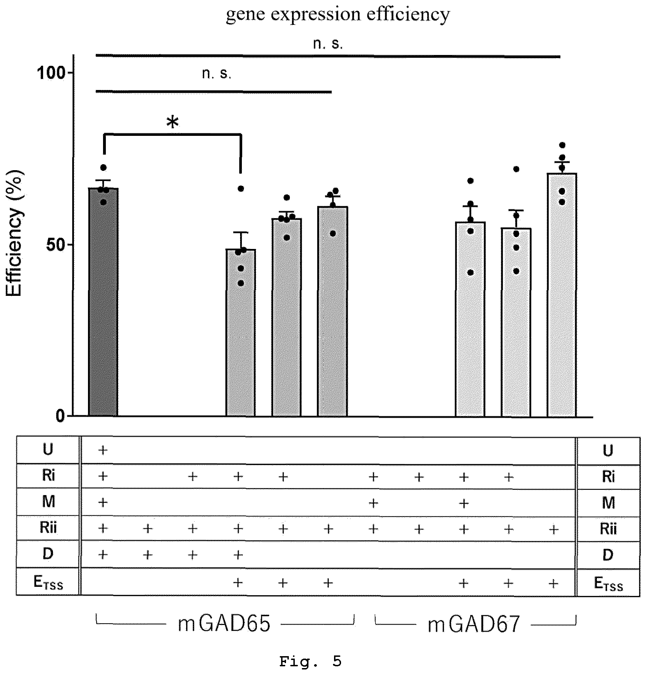

The results of quantitative analysis of gene expression efficiency are shown in Figure 5 (Mean ± SEM (standard error) (n = 4 to 5), n.s.: Not Significant, *: P < 0.05, 1-way ANOVA, Dunnett's test). It was shown that 64.7% ± 0.99% of cells expressing GFP under the control of the original mGAD65 promoter were do-labeled with tdTomato (n = 4 to 5). In addition, it was shown that the specificity of gene expression was 61.4% ± 2.42% when the shortest cmGAD65 among the shortened mGAD65 promoters (namely, mGAD65 (Rii-E_{TSS})) was used, and the specificity of gene expression was 71.3% ± 2.74 when the shortest cmGAD67 among the shortened mGAD67 promoters (namely, mGAD67 (Rii-E_{TSS})) was used.

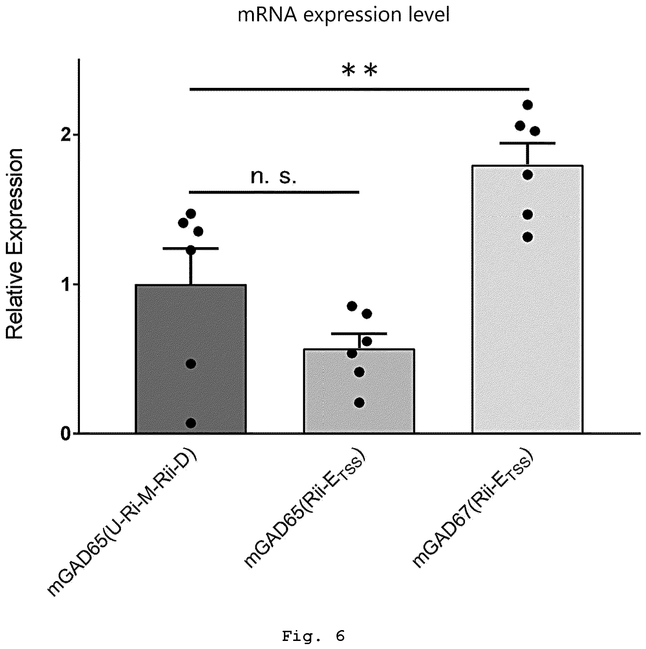

Moreover, Figure 6 shows the mRNA expression level when the experiment of gene expression in the cerebral cortex using various shortened mGAD promoters was carried out (Mean ± SEM (standard error) (n = 6), n.s.: Not Significant, *: P < 0.01, 1-way ANOVA, Dunnett's test). The results showed that the mRNA expression level was significantly increased in mGAD67 (Rii-E_{TSS}) compared with mGAD65 (U-Ri-M-Rii-D).

Three weeks after injection of AAV-PHP.eB expressing GFP under the designed shortened mGAD65 or 67 promoter, mRNA was isolated from cerebral cortex tissues of VGAT-tdTomato mice using RNeasy Mini Kits (QIAGEN, Hilden, Germany). Reverse transcription was performed using ReverTra Ace quantitative reverse transcriptase-polymerase chain reaction (RT-PCR) Master Mix with gDNA Remover (TOYOBO Co., Ltd., Osaka, Japan), cDNA was synthesized from the cerebral cortex mRNA samples, and real-time quantitative PCR was performed using Power SYBR Green qPCR Mix (TOYOBO Co., Ltd., Osaka, Japan). The forward (F) and reverse (R) primers used (GAPDH and GFP) had the following sequences: qRTPCR-mGAPDH-F: 5'-GTGTTCCTACCCCCAATGTG-3'(SEQ ID No. 47); qRTPCR-mGAPDH-R: 5'-GGTGGAAGAGTGGGAGTTGCTG-3'(SEQ ID No. 48); qPCR-AAV-EGFP-F: 5'-GGACGAGCTGTACAAGTAAAG-3'(SEQ ID No. 49); qPCR-AAV-WPRE-R: 5'-GGGAAGCAATAGCATGATACAAAGG-3'(SEQ ID No. 50). The thermal cycling program used in real-time quantitative PCR was carried out with an initial denaturation step at 95°C for 10 min, followed by 40 cycles of 95°C for 15 sec and 60°C for 1 min. The obtained data was analyzed by the ΔΔCt method, and the expression levels in the cmGAD65 and cmGAD67 promoters were shown when the GFP expression level in the mGAD65 (U-Ri-M-Rii-D) promoter was set to 1.

From the above, it was demonstrated that the mGAD65 promoter, which was as long as 2.5 kb, could be shortened as disclosed in the present invention, and in particular, in the case of using cmGAD65 or cmGAD67, it could be made compact to as small as 0.5 kb. Furthermore, in the case of cmGAD67, the promoter activity could be increased by about two-fold.

## Claims

1. A promoter consisting of DNA having a promoter activity, comprising:
a base sequence of a Dlx1 binding site and/or a Dlx2 binding site in a glutamic acid decarboxylase (GAD) promoter; and
a base sequence of a region ranging from a 5' end of exon 1 to a transcription start site (TSS) (E_{TSS} region) in a glutamic acid decarboxylase (GAD).

2. The promoter according to claim 1, wherein
the glutamic acid decarboxylase (GAD) promoter is a mouse-derived GAD65 promoter (mGAD65 promoter),
the glutamic acid decarboxylase (GAD) is a mouse-derived glutamic acid decarboxylase 65 (mGAD65),
the base sequence of the Dlx1 binding site and/or the Dlx2 binding site includes at least one base sequence selected from a base sequence of Region i of the mGAD65 promoter represented by SEQ ID No. 1, a base sequence of Region ii of the mGAD65 promoter represented by SEQ ID No. 2, and a base sequence that is at least 90% identical to any of these base sequences, and
the base sequence of the region ranging from the 5' end of exon 1 to the transcription start site (TSS) (E_{TSS} region) in the mGAD65 is a base sequence represented by SEQ ID No. 3 or a base sequence that is at least 90% identical to the base sequence.

3. The promoter according to claim 1, comprising a base sequence represented by any one of SEQ ID Nos. 4 to 6 or a base sequence that is at least 90% identical to the base sequence represented by any one of SEQ ID Nos. 4 to 6.

4. The promoter according to claim 1, wherein
the glutamic acid decarboxylase (GAD) promoter is a mouse-derived GAD67 promoter (mGAD67 promoter),
the glutamic acid decarboxylase (GAD) is a mouse-derived glutamic acid decarboxylase 67 (mGAD67),
the base sequence of the Dlx1 binding site and/or the Dlx2 binding site includes at least one base sequence selected from a base sequence of Region i of the mGAD67 promoter represented by SEQ ID No. 7, a base sequence of Region ii of the mGAD67 promoter represented by SEQ ID No. 8, and a base sequence that is at least 90% identical to any of these base sequences, and
the base sequence of the region ranging from the 5' end of exon 1 to the transcription start site (TSS) (E_{TSS} region) in the mGAD67 is a base sequence represented by SEQ ID No. 9 or a base sequence that is at least 90% identical to the base sequence.

5. The promoter according to claim 1, comprising a base sequence represented by any one of SEQ ID Nos. 10 to 12 or a base sequence that is at least 90% identical to the base sequence represented by any one of SEQ ID Nos. 10 to 12.

6. An adeno-associated viral vector or a recombinant virus obtained therefrom for gene expression in an inhibitory neuron, comprising the promoter according to any one of claims 1 to 5.

7. The adeno-associated viral vector or the recombinant virus obtained therefrom according to claim 6, further comprising a target gene arranged under the control of the promoter.

8. A medicine, comprising the adeno-associated viral vector or the recombinant virus obtained therefrom according to claim 7.

9. The medicine according to claim 8, for treating a central nervous system disease caused by a dysfunction of inhibitory neurons or an abnormal balance of excitation and inhibition.

10. A method for transfecting a gene into a cell, comprising a step of transfecting a target gene into a target cell *in vitro* by using the adeno-associated viral vector or the recombinant virus obtained therefrom according to claim 7.

11. The method according to claim 10, wherein the target cell is an inhibitory neuron.
